# FASCICULE DE BREVET EUROPEEN

(11) **EP 0 853 472 B1**
(45) Date de publication et mention de la délivrance du brevet: **23.04.2003**
(21) Numéro de dépôt: 96932674.3
(22) Date de dépôt: 30.09.1996
(51) Int. Cl.: A61K 7/48

(54) **UTILISATION DE CARBOHYDRATES POUR FAVORISER LA DESQUAMATION DE LA PEAU**
VERWENDUNG VON KOHLEHYDRATEN ZUR FOERDERUNG DER SCHUPPENDEN HAUT
USE OF CARBOHYDRATES FOR PROMOTING SKIN EXFOLIATION

(30) Priorité: 04.10.1995 FR 9511661
(43) Date de publication de la demande: 22.07.1998
(73) Titulaire: L'OREAL, 75008 Paris (FR)
(72) Inventeur: PHILIPPE, Michel, F-91320 Wissous (FR); EBENHAN-NAPPE, Catherine, F-93600 Aulnay-sous-Bois (FR)
(74) Mandataire: Corizzi, Valérie
(86) Numéro de dépôt international: FR9601522
(87) Numéro de publication internationale: WO97012597

(56) Documents cités:
- WO-A-95/05155
- FR-A- 2 703 993

## Description

L'invention se rapporte à l'utilisation de dérivés carbohydrates dans ou pour la fabrication d'une composition cosmétique et/ou dermatologique pour favoriser la desquamation de la peau et/ou lutter contre le vieillissement intrinsèque et extrinsèque de la peau. Elle se rapporte aussi à un procédé de traitement non thérapeutique de la peau en vue de desquamer la peau ainsi qu'à un procédé non thérapeutique de traitement du vieillissement cutané.

Le vieillissement cutané résultant d'effets sur la peau de facteurs intrinsèques ou extrinsèques, se traduit par l'apparition de rides et ridules, par le jaunissement de la peau qui développe un aspect parcheminé accompagné de l'apparition de taches pigmentaires, par la désorganisation des fibres d'élastine et de collagène entraînant une perte d'élasticité, de souplesse et de fermeté et par l'apparition de télangiectasies.

Certains de ces signes du vieillissement sont plus particulièrement liés au vieillissement intrinsèque ou physiologique, c'est-à-dire au vieillissement «normal» lié à l'âge, alors que d'autres sont plus spécifiques du vieillissement extrinsèque, c'est-à-dire du vieillissement provoqué d'une manière générale par l'environnement ; il s'agit plus particulièrement du photo-vieillissement dû à l'exposition au soleil, à la lumière ou à tout autre rayonnement.

L'invention s'intéresse au vieillissement intrinsèque ou physiologique ainsi qu'au vieillissement extrinsèque.

Les changements de la peau dus au vieillissement intrinsèque sont la conséquence d'une sénescence génétiquement programmée où interviennent des facteurs endogènes. Ce vieillissement intrinsèque provoque notamment un ralentissement du renouvellement des cellules de la peau, ce qui se traduit essentiellement par l'apparition d'altérations cliniques telles que la réduction du tissu adipeux sous-cutané et l'apparition de fines rides ou ridules, et par des changements histopathologiques tels qu'une augmentation du nombre et de l'épaisseur des fibres élastiques, une perte de fibres verticales de la membrane du tissu élastique, et la présence de grands fibroblastes irréguliers dans les cellules de ce tissu élastique.

Au contraire, le vieillissement extrinsèque entraîne des altérations cliniques telles que des rides épaisses et la formation d'une peau molle et tannée, et des changements histopathologiques tels qu'une excessive accumulation de matière élastique dans le derme supérieur et une dégénérescence des fibres de collagène.

On connaît dans l'art antérieur divers agents destinés à lutter contre le vieillissement cutané.

Ainsi, le brevet US-A-4 603 146 décrit l'emploi d'acide rétinoïque et de ses dérivés dans des compositions cosmétiques, en vue de lutter contre le vieillissement cutané.

Par ailleurs, de nombreux brevets et publications (voir par exemple la demande EP-A- 413 528) ainsi que de nombreuses compositions cosmétiques du commerce enseignent l'emploi des α-hydroxyacides comme l'acide lactique, l'acide glycolique ou encore l'acide citrique pour traiter le vieillissement cutané.

On connaît enfin les bêta-hydroxy-acides et plus spécialement l'acide salicylique ainsi que ses dérivés pour leurs propriétés desquamantes (voir les documents WO-A-93/10756 et US-A-4 767 750).

Tous ces composés ont une action contre le vieillissement de la peau, consistant en une desquamation, c'est-à-dire l'élimination des cellules « mortes » situées à la surface du *stratum corneum.* Cette propriété desquamante est aussi appelée, souvent à tort, propriété kératolytique. Mais ces composés présentent également des effets secondaires, qui consistent en des picotements, des tiraillements, des échauffements et des rougeurs désagréables pour l'utilisateur.

On constate donc que subsiste le besoin d'agents antivieillissement ayant une action au moins aussi efficace que celle des composés de l'art antérieur, mais ne présentant pas leurs inconvénients.

Par ailleurs, Brysk (Cell and tissue research 253, 657-663, 1988 ; Expl. Cell Biol. 57, 60-66, 1989) a montré le rôle des glycoprotéines dans la cohésion du *stratum corneum*. Elle a également mis en évidence l'action inhibitrice de certains carbohydrates, en particulier des carbohydrates aminés, vis-à-vis de la cohésion du *stratum corneum.*

La demanderesse a découvert de manière inattendue que certains dérivés carbohydrates montraient une activité inhibitrice de la cohésion du *stratum corneum* très importante, supérieure à l'action des dérivés analogues déjà connus pour cette activité.

Par conséquent, l'application topique de ces nouveaux dérivés permet de desquamer la peau et de lutter contre le vieillissement cutané.

Certes, il est connu du document US-A-5084270 d'utiliser en application topique, pour le traitement des peaux sèches, des amides issues de la condensation d'un carbohydrate acide et d'une amine primaire. Toutefois, il n'est ni mentionné ni suggéré dans l'art antérieur une action desquamante de ces produits sur la peau.

Le document WO95/05155 décrit des dérivés lipophiles des sucres, leur utilisation dans un véhicule cosmétique comme modulateurs de la synthèse et/ou de l'excrétion de l'élastase des fibroblastes. Toutefois, il n'est ni mentionné, ni suggéré dans ce document une action desquamante de dérivés carbohydrates.

La présente invention a pour objet l'utilisation dans ou pour la fabrication d'une composition topique, dans les domaines cosmétique, dermatologique et/ou pharmaceutique, d'au moins un carbohydrate ou dérivé de carbohydrate répondant à la formule (I),

R-X-A (I)

dans laquelle
A représente une chaîne composée de une à vingt unités carbohydrate ou dérivé de carbohydrate, comprenant chacune 3 à 6 atomes de carbone, reliées entre elles, de préférence par des ponts acétals, chacune de ces unités pouvant être éventuellement substituée, par exemple par un halogène, par une fonction amine, une fonction acide, une fonction ester, un thiol, une fonction alcoxy, une fonction thio-éther, une fonction thio-ester, une fonction amide,une fonction carbamate, une fonction urée,
R représente une chaîne alcoyle ou une chaîne alcényle, comprenant de 4 à 24 atomes de carbone, ramifiée ou linéaire, pouvant être interrompue par des ponts éthers, portant éventuellement une fonction hydroxyle, une fonction acide carboxylique, une fonction amine, une fonction ester, une fonction acyloxy, une fonction amide, une fonction éther, une fonction carbamate, une fonction urée,
X représente une fonction reliant R et A, comme par exemple une fonction amine, éther, amide, ester, urée, carbamate, thioester, thioéther, sulfonamide,
pour favoriser la desquamation de la peau.

L'invention a également pour objet l'utilisation de carbohydrates tels que décrits ci-dessus pour lutter contre le vieillissement cutané.

De façon préférentielle, R représente une chaîne alcoyle ou une chaîne alcényle, comprenant de 4 à 24 atomes de carbone, ramifiée ou linéaire, portant éventuellement une fonction hydroxyle.

Chacune des unités carbohydrate composant A peut être un sucre ou un dérivé de sucre. Par exemple, chaque unité composant A peut être un sucre réduit, un sucre aminé ou un sucre porteur d'une fonction acide carboxylique.

Parmi les sucres, ou les dérivés de sucre, pouvant entrer dans la constitution de A, on citera par exemple les produits suivants, qui sont disponibles commercialement, éventuellement sous forme de sel : la N-acétyl-D-galactosamine, la N-acétyl-D-glucosamine, l'acide N-acétyl-neuraminique, l'adonitol, le β-D-allose, le D-altrose, le 6-amino-6-déoxy-D-glucose, le 1,6-anhydroglucose, l'acide arabinique, l'arabinogalactan, le D-arabinose, le L-arabinose, le D,L-arabinose, le D-arabitol, le L-arabitol, le D-cellobiose, la D-glucosamine, la D-galactosamine, le 2-déoxy-D-glucose, le 6-déoxy-D-galactose, le 6-déoxy-L-galactose, le galactitol, le mésoérythritol, le D-érythrose, le D-fructose, le D-fucose, le L-fucose, l'acide D-galactarique, le galactitol, le galactomannane, le D-galactono-1,4-lactone, le L-galactono-1,4-lactone, la D-galactosamine, le D-galactose, le L-galactose, l'acide D-galacturonique, le β-gentiobiose, la glucamine, l'acide D-glucarique, l'acide D-glucarique, le D-glucono-1,5-lactone, le L-glucono-1,5-lactone, la D-glucosamine, l'acide D-glucosaminique, l'acide D-glucuronique, le L-glucose, le D-glucose, l'isomaltitol, l'isomaltotriose, l'isomaltose, l'acide lactobionique, le D-lactose, le lactulose, le D-lyxose, le L-lyxose, la lyxosamine, le maltitol, le D-maltose, le maltotétraose, le maltotriitol, le maltotriose, la D-mannosamine, le D-mannose, le L-mannose, le D-mélézitose, le D-mélibiose, le D-raffinose, l'undeca-acétate de D-raffinose, le L-rhamnose, le D-ribose, le L-ribose, le D-ribulose, le rutinose, le D-saccharose, l'α-sophorose, le sorbitol, le D-tagatose, le D-talose, le D-thréose, le turanose, le D-xylitol, le D-xylose, le L-xylose, le D, L-xylose.

De façon préférentielle A sera choisie parmi les chaînes hydrocarbonées suivantes :
la D-glucosamine ou 2-amino-2-déoxy-D-glucose, la D-glucamine ou 1-amino-1-déoxy-D-glucitol, la N-méthyl-glucamine, le D-glucose, le D-maltose, le sorbitol, le maltitol.

De façon préférentielle R comprend 4 à 16 atomes de carbone comme par exemple les radicaux n-butyle, n-octyle, 2-éthyl-hexyle, n-dodécyle.

Selon l'invention, les compositions préférées comprendront au moins un produit choisi parmi :
le N-butanoyl-D-glucosamine, le N-octanoyl-D-glucosamine, le N-octyloxycarbonyl-N-méthyl-D-glucamine, le N-2-éthyl-hexyloxycarbonyl-N-méthyl-D-glucamine, le 6-O-octanoyl-D-glucose, le 6'-O-octanoyl-D-maltose, le 6'-O-dodécanoyl-D-maltose.

La préparation de produits (I) est bien connue de l'homme du métier, on pourra par exemple se référer aux documents suivants : FR-A-2703993, FR-A-2715933, EP-A-577506, EP-A-566438, EP-A-485251.

Dans les compositions selon l'invention, le carbohydrate selon (I) ou le mélange de carbohydrates selon (I) peut être utilisé en une quantité allant de 0,05 à 20 % en poids par rapport au poids total de la composition et en particulier en une quantité allant de 0,2 à 10 % et mieux de 0,5 à 5 % en poids par rapport au poids total de la composition.

Dans les compositions que l'on peut utiliser selon l'invention, les carbohydrates répondant à la formule (I) peuvent être associés à d'autres actifs ayant des propriétés desquamantes, comme les hydroxy-acides, les α- ou β-céto-acides, les rétinoïdes, certains acides sulfoniques. Une telle association permet de diminuer la concentration active de ces derniers du fait des effets additifs. On peut ainsi obtenir une composition moins irritante et moins toxique ainsi qu'une composition plus efficace que celles de l'art antérieur n'utilisant que ces actifs.

Les hydroxyacides peuvent être par exemple des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Ces hydroxyacides sont notamment les acides glycolique, lactique, malique, tartrique, citrique et de manière générale les acides de fruits, les acides hydroxy-2 alcanoïque, mandélique, salicylique, ainsi que leurs dérivés alkylés ou acylés comme l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque ou encore leurs dérivés alcoxylés comme l'acide 2-hydroxy-3-méthoxybenzoïque.

Les rétinoïdes peuvent être notamment l'acide rétinoïque (all-trans ou 13-cis) et ses dérivés, le rétinol (vitamine A) et ses esters tels que le palmitate de rétinol, l'acétate de rétinol et le propionate de rétinol ainsi que leurs sels, ou encore le rétinal.

A titre d'exemple, les hydroxyacides, les céto-acides et les rétinoïdes peuvent être introduits dans les compositions utilisées selon l'invention en une quantité représentant de 0,01 à 5 % en poids du poids total de la composition et mieux de 0,1 à 3 %.

En vue de lutter efficacement contre le photovieillissement, il est en outre possible d'ajouter à la composition utilisée selon l'invention un ou plusieurs filtres solaires complémentaires, actifs dans l'UVA et/ou l'UVB, hydrophiles ou lipophiles, comportant éventuellement une fonction sulfonique.

Un test pour mesurer l'efficacité des carbohydrates a été réalisé in vitro.

Brysk (Cell and tissue research 253, 657-663, 1988 ; Differentiation, 32, 230-237, 1986) a montré que l'hémagglutination constituait un modèle fiable pour l'étude de la cohésion cornéocytaire : Elle a mis en évidence une hémagglutination due à une glycoprotéine du *stratum corneum*, ainsi qu'une inhibition par les mêmes sucres aminés à la fois de l'hémagglutination et de la cohésion du *stratum corneum.*

Le principe du test repose sur le fait que la lectine provoque l'agglutination des érythrocytes.

Les produits à tester, préparés à des dilutions variées, sont additionnés aux solutions de lectine. On mesure la concentration minimale de produit permettant d'inhiber l'hémagglutination provoquée par la lectine.

Les produits les plus efficaces sont ceux montrant une activité inhibitrice à la plus faible concentration possible.

Les résultats des tests montrent une activité des produits utilisés selon l'invention, à des concentrations bien inférieures aux concentrations actives minimales des produits de l'état de l'art testés à titre de référence (voir plus loin).

L'invention a encore pour objet un procédé de traitement cosmétique ou dermatologique de la peau destiné à la desquamation de la peau consistant à appliquer sur la peau une composition contenant au moins un carbohydrate de formule (I), dans un milieu cosmétiquement et/ou dermatologiquement acceptable.

L'invention a aussi pour objet un procédé de traitement cosmétique ou dermatologique du vieillissement de la peau, consistant à appliquer sur la peau une composition contenant au moins un carbohydrate tel que défini ci-dessus, dans un milieu cosmétiquement et/ou dermatologiquement acceptable.

La composition utilisée selon l'invention contient un milieu cosmétiquement ou dermatologiquement acceptable, c'est-à-dire un milieu compatible avec la peau, les ongles, les muqueuses, les tissus et les cheveux. La composition contenant un ou plusieurs carbohydrate selon (I) peut être appliquée par voie topique sur le visage, le cou, les cheveux, les muqueuses et les ongles ou toute autre zone cutanée du corps.

Les compositions utilisées selon l'invention peuvent se présenter sous toutes les formes appropriées pour une application topique, notamment sous forme de solutions aqueuses, hydroalcooliques ou huileuses, de dispersions du type lotion ou sérum, de gels aqueux, anhydres ou huileux, d'émulsions de consistance liquide ou semi-liquide du type lait, obtenues par dispersion d'une phase grasse dans une phase aqueuse (H/E) ou inversement (E/H), de suspensions ou d'émulsions de consistance molle, semi-solide ou solide du type crème, gel, de microémulsions, ou encore de microcapsules, de microparticules, ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions utilisées selon l'invention sont celles classiquement utilisées dans les domaines considérés.

Ces compositions constituent notamment des crèmes de protection, de traitement ou de soin pour le visage, pour les mains ou pour le corps, des laits corporels de protection ou de soin, des lotions, gels ou mousses pour le soin de la peau et des muqueuses ou pour le nettoyage de la peau.

Les compositions peuvent également consister en des préparations solides constituant des savons ou des pains de nettoyage.

De façon connue, la composition utilisée selon l'invention peut contenir également des adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (huile de karité, huile d'amande douce), les huiles animales, les huiles de synthèse, les huiles siliconées (cyclométhicone) et les huiles fluorées (perfluoropolyéthers). On peut aussi utiliser comme matières grasses des alcools gras, des acides gras (acide stéarique), des cires (paraffine, camauba, cire d'abeilles).

Comme émulsionnants utilisables dans l'invention, on peut citer le Polysorbate 60 et le stéarate de sorbitane vendus respectivement sous les dénominations commerciales Tween 60 et Span 60 par la Société ICI. On peut y ajouter des co-émulsionnants tels que le PPG-3 myristyl éther vendu sous la dénomination commerciale Emcol 249-3K par la société Witco.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles (xanthane) et les argiles, et, comme gélifiants lipophiles, on peut citer les argiles modifiées comme les bentones, les sels métalliques d'acides gras comme les stéarates d'aluminium, la silice hydrophobe, les polyéthylènes et l'éthylcellulose.

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon, les extraits bactériens ou végétaux, notamment d'Aloe Vera.

Comme actifs lipophiles, on peut utiliser le tocophérol (vitamine E) et ses dérivés, les acides gras essentiels, les céramides, les huiles essentielles.

On peut, entre autre, associer les carbohydrates selon l'invention à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que la vitamine D et ses dérivés, les oestrogènes tels que l'oestradiol, l'acide kojique ou l'hydroquinone ;
- les agents anti-radicaux libres, tels que l'alpha-tocophérol ou ses esters, les superoxyde dismutases, certains chélatants de métaux ou l'acide ascorbique et ses esters.

On peut, par ailleurs, associer aux carbohydrates de l'invention des antagonistes de substance P et/ou de CGRP (Calcitonin Gene Related Peptide ou peptide lié au gène de la calcitonine) tels que l'Iris Pallida et les sels de strontium, notamment les chlorures et les nitrates de strontium, ou des antagonistes de substance P et/ou de CGRP tels que ceux décrits dans les demandes de brevet français déposées au nom de la demanderesse sous les numéros 9405537 et 9500900. Une telle association permet de garantir une tolérance parfaite de ces compositions, même par des peaux très sensibles.

Le procédé de traitement cosmétique ou dermatologique de l'invention peuvent être mis en oeuvre notamment en appliquant les compositions hygiéniques, cosmétiques ou dermatologiques telles que définies ci-dessus, selon la technique d'utilisation habituelle de ces compositions. Par exemple : application de crèmes, de gels, de sérums, de pommades, de lotions, de laits sur la peau, le cuir chevelu, les ongles et/ou les muqueuses.

Les exemples suivants illustrent l'invention. Dans ces exemples, les proportions indiquées sont des pourcentages en poids.

### Exemples

### Exemple 1 : Préparation du 6'-0-octanoyl-β-D-maltose

### A-Activation de l'acide octanoïque

Dans un ballon de 500ml muni d'un système d'agitation, d'un thermomètre, d'un réfrigérant ascendant avec une garde à chlorure de calcium, on introduit 12,6g (0,103 mole) de chloroformiate d'isopropyle et 100ml de tétrahydrofurane. Au mélange agité et refroidi à -10°C on additionne goutte à goutte une solution constituée de 14,4g (0,1mole) d'acide octanoïque, de 10,4g (0,103mole) de triéthylamine dissouts dans 100ml de tétrahydrofurane. Pendant l'addition, la température est maintenue entre -10°C et -15°C, puis, à l'issue, on laisse le mélange revenir à température ambiante et on élimine les sels de triéthylamine par filtration.

### B-Préparation du 6'-O-octanoyl-β-D-maltose:

Dans un ballon de 2litres muni d'une agitation, d'un réfrigérant ascendant, on introduit 108g (0,3 mole) de maltose monohydraté que l'on dissout dans 540ml de pyridine. A ce mélange on additionne la solution d'acide octanoïque activé préparée dans A, et on laisse sous agitation à température ambiante pendant 17 heures. Le mileu réactionnel est alors concentré sous vide puis repris par un mélange de solvants (150ml d'acétate d'éthyle, 150ml d'heptane, 300ml d'eau). On laisse le milieu décanter, la phase aqueuse est isolée puis lavée deux fois à l'aide de 250ml d'un mélange acétate d'éthyle/heptane: 1/2. Enfin le 6'-O-octanoyl-D-maltose est extrait à l'aide d'un mélange acétate d'éthyle/butanol: 2/1. Après évaporation des solvants on récupère 13,1g de 6'-O-octanoyl-β-D-maltose.
rendement : 28%
point de fusion : 226°C
les spectres RMN H¹ et C¹³ sont en conformité avec la structure du produit.

| analyse élémentaire : | | | |
|---|---|---|---|
| | C | H | O |
| calculé | 51,3 | 7,7 | 41 |
| trouvé | 51,2 | 7,8 | 40,9 |

### Exemple 2 : Préparation du 6-O-octanoyl-α-D-glucose

Dans un ballon de 2litre muni d'une agitation, d'un réfrigérant ascendant, on introduit 72g (0,4 mole) de glucose anhydre que l'on dissout dans 860ml de pyridine.
A ce mélange on additionne la solution d'acide octanoïque activé préparée dans l'exemple 1-A, et on laisse sous agitation à température ambiante pendant 17 heures. Le mileu réactionnel est alors concentré sous vide puis repris par un mélange d'eau et d'acétonitrile et lavé trois fois par un mélange acétate d'éthyle/heptane : 1/1.
La phase aqueuse est isolée et le 6-O-octanoyt-α-D-glucose est extrait à l'aide d'un mélange acétate d'éthyle/butanol : 2/1. Après évaporation des solvants, le milieu est repris dans 60ml d'acétonitrile chaud. Le 6-O-octanoyl-α-D-glucose cristallise lorsqu'on laisse la solution revenir à température ambiante. Par filtration et séchage, on récupère 14,6g de 6'-O-octanoyl-α-D-glucose.
rendement : 48%
point de fusion : 128°C
les spectres RMN H¹ et C¹³ sont en conformité avec la structure du produit.

| analyse élémentaire : | | | |
|---|---|---|---|
| | C | H | O |
| calculé | 54,9 | 8,5 | 36,6 |
| trouvé | 55,1 | 8,5 | 36,3 |

### Exemple 3 : Préparation de la N-octyloxycarbonyl-N-méthvl-D-glucamine

Dans un réacteur de 1 litre on dissout sous agitation 24,37g (0,125mole) de N-méthyl-D-glucamine dans 650ml d'eau et 850ml de tétrahydrofurane, puis on ajoute 42g de bicarbonate de sodium (0,5mole) et on amène la température du milieu à 5°C. On ajoute au goutte à goutte, 24,06g (0,125 mole) de chloroformiate d'octyle, en maintenant le milieu à 5°C pendant une heure après l'addition. Le milieu est ramené à température ambiante, filtré, décanté. La phase organique est récupérée, le solvant évaporé sous vide et le résidu repris par 1,5l d'acétone au reflux. La. N-octyloxycarbonyl-N-méthyl-D-glucamine précipite à froid. On récupère par filtration puis séchage 24g de produit.
rendement : 55%
point de fusion : 128°C
les spectres RMN H¹ et C¹³ sont en conformité avec la structure du produit.

| analyse élémentaire: N-octyloxycarbonyl-N-méthyl-D-glucamine, ½ H₂O | | | | |
|---|---|---|---|---|
| | C | H | N | O |
| calculé | 53,3 | 9,5 | 3,9 | 33,3 |
| trouvé | 53,3 | 9,6 | 3,9 | 33,2 |

### Exemple 4 : Préparation de la N-2-éthyl-hexyloxycarbonyl-N-méthyl-D-glucamine

On procède comme à l'exemple 3 à partir de 0,125 mole de chloroformiate de 2-éthyl-hexyloxycarbonyl préparé suivant l'exemple 1-A.
rendement : 58%
point de fusion : 77°C
les spectres RMN H¹ et C¹³ sont en conformité avec la structure du produit.

| analyse élémentaire : | | | | |
|---|---|---|---|---|
| | C | H | N | O |
| calculé | 54,7 | 9,5 | 4,0 | 31,8 |
| trouvé | 54,5 | 9,6 | 4,0 | 31,7 |

### Exemple 5 : Préparation de la N-butanoyl-D-glucosamine

15g de chlorhydrate de D-glucosamine sont solubilisés dans 150ml de méthanol, à température ambiante. Après addition d'un équivalent de méthylate de sodium et filtration du chlorure de sodium engendré, 14,8ml d'anhydride butyrique sont ajoutés progressivement au milieu réactionnel qui est agité ensuite pendant 3 heures à température ambiante. Un précipité se forme qui est recueilli par précipitation puis lavé et recristallisé dans 130ml d'éthanol chaud. On récupère ainsi 8,8g de N-butanoyl-D-glucosamine.
rendement : 51%
point de fusion : 212°C
les spectres RMN H¹ et C¹³ sont en conformité avec la structure du produit.

| analyse élémentaire : | | | | |
|---|---|---|---|---|
| | C | H | N | O |
| calculé | 48,2 | 7,7 | 5,6 | 38,5 |
| trouvé | 48,4 | 7,8 | 5,7 | 38,3 |

### Tests :

Pour la réalisation des tests on emploie des érythrocytes de lapin, commercialisés par la société Biomérieux sous la référence 72291. Ces érythrocytes sont utilisés en suspension à 50% dans l'eau. La lectine utilisée est Banderia simplicifolia isolectine B4, commercialisée par la société SYGMA.

Toutes les solutions sont diluées dans du tampon phosphate contenat du calcium et du magnésium.

### A- Hémagglutination par la lectine :

La lectine est mise en solution dans le tampon phosphate à la concentration de 1mg/ml. Dans tous les puits des plaques de microtitration sont distribués 25µl de tampon phosphate. Dans le second puits, on ajoute 25µl de la lectine à tester, Après homogénéisation, 25µl de ce puits sont transférés dans le puits suivant et ainsi de suite jusqu'à obtenir des dilutions successives. Les érythrocytes sont dilués au 1/10^{e} (solution fraîche). Sur lame, on dépose 5µl de la suspension et 5µl du contenu d'un puits. On observe après mélange par rotation s'il y a ou non hemagglutination. Celle-ci se développe en 5min au maximum. On mesure ainsi la quantité minimale de lectine permettant d'obtenir une hémagglutination érythrocytaire nette.

### B- Inhibition de l'hémagglutination :

Après avoir mesuré la quantité minimale de lectine permettant d'obtenir une hémagglutination érythrocytaire nette, on choisit de travailler à une concentration double. Dans des plaques de microtitration, on place 25µl de tampon phosphate pour réaliser un témoin négatif. Dans les autres puits on distribue 25µl de lectine à la concentration déterminée. Dans le second puits, on ajoute 25µl de tampon phosphate pour obtenir un témoin positif. Dans le troisième puits, on place 25µl du dérivé carbohydrate à tester en solution à la concentration de 0,1M Après homogénéisation, on reprend 25µl de ce puits que l'on place dans le suivant et ainsi de suite de façon à obtenir des dilutions successives. Après avoir laissé la lectine et le dérivé carbohydrate pendant 30min en contact à température ambiante, on place 5µl sur une lame que l'on mélange avec 5µl de suspension érythrocytaire. On observe s'il y a ou non hemagglutination après agitation par rotation.

### C- Résultats :

On mesure la dilution de carbohydrate la plus basse permettant d'observer une inhibition de l'hémagglutination. Le résultat est donné en fraction (correspondant à la dilution) de la concentration initiale (0,1M).
1) Produits selon l'invention :
   6'-O-octanoyl-D-maltose : 1/4
   N-2-éthyl-hexyloxycarbonyl-N-méthyl-D-glucamine : 1/16
   N-octyloxycarbonyl-N-méthyl-D-glucamine : 1/64
2) Produit selon l'art antérieur :
   N-acétyl-D-glucosamine : pas d'inhibition observée pour la solution non dilué (concentration : 0,1M).

On peut donc conclure de ces résultats que les composés selon l'invention sont plus efficaces que les produits décrits dans l'art antérieur (Brysk, Cell and tissue research 253, 657-663, 1988).

### Exemples de composition :

Ces exemples illustrent l'invention. Les proportions indiquées sont des pourcentages en poids.

### Composition1 : Emulsion H/E

### Phase A :

- 6'-O-octanoyl-D-maltose 2,5
- Huile d'amande douce 14,5
- Huile de karité 7,0
- PPG-3 myristyl éther (EMCOL 249-3K) 5,0
- Conservateur (propylparabène) 0,1
- Polysorbate 60 (TWEEN 60) 2,5
- Stéarate de sorbitane (SPAN 60) 2,5

### Phase B :

- Cyclométhicone 4,0
- Gomme de xanthane 0,2
- Polymère carboxyvinylique 0,5

### Phase C :

- Triéthanolamine (neutralisant) 0,5
- Eau 2,0

### Phase D :

- Conservateur (méthylparabène) 0,2
- Glycérine 5,0
- Eau qsp 100

### Mode opératoire :

On fait fondre les constituants de la phase A à 85° C, puis on refroidit la phase A à 70° C et on y introduit les phases B puis C et D sous agitation. On refroidit jusqu'à la température ambiante. On obtient une crème de jour qui provoque la desquamation de la peau et confère ainsi à celle-ci un aspect plus lisse et plus jeune qu'avant le traitement.

### Composition 2 : Gel

- N-2-éthyl-hexyloxycarbonyl-N-méthyl-D-glucamine 5,0
- Hydroxypropylcellulose (Klucel H de la société Hercules) 1,0
- Antioxydant 0,05
- Isopropanol 40,0
- Conservateur 0,3
- Eau qsp 100

On obtient un gel qui, en application régulière, permet d'estomper les taches de la peau par desquamation.

### Composition 3 : Solution pour application dermatologique

- N-octyloxycarbonyl-N-méthyl-D-glucamine 5,00
- Antioxydant 0,05
- Alcool éthylique 10,00
- Conservateur 0,30
- Eau qsp 100

L'application sous contrôle dermatologique de cette solution permet d'obtenir une desquamation profonde de la couche cornée et, ainsi, la mise en jeu d'un processus de réparation épidermique, ayant comme effet thérapeutique final un effacement des taches et dyschromies, un estompement des rides et ridules et une amélioration de l'état clinique de la peau, dont l'aspect devient celui d'une peau plus jeune.

Cette application se fait à raison d'une à trois séances hebdomadaires pendant 4 à 6 semaines.

## Revendications

1. Utilisation pour la fabrication d'une composition topique destinée à favoriser la desquamation de la peau, d'au moins un carbohydrate ou dérivé de carbohydrate répondant à la formule (I),
R-X-A (I)
dans laquelle
A représente une chaîne composée de une à vingt unités carbohydrate, comprenant chacune 3 à 6 atomes de carbone, reliées entre elles, chacune de ces unités pouvant être éventuellement substituée par un halogène, par une fonction amine, une fonction acide, une fonction ester, un thiol, une fonction alcoxy, une fonction thio-éther, une fonction thio-ester, une fonction amide, une fonction carbamate, une fonction urée,
R représente une chaîne alcoyle ou une chaîne alcényle, comprenant de 4 à 24 atomes de carbone, ramifiée ou linéaire, pouvant être interrompue par des ponts éthers, portant éventuellement une fonction hydroxyle, une fonction acide carboxylique, une fonction amine, une fonction ester, une fonction acyloxy, une fonction amide, une fonction éther, une fonction carbamate, une fonction urée,
X représente une fonction susceptible de relier R et A.

2. Utilisation dans une composition topique, d'au moins un carbohydrate ou dérivé de carbohydrate répondant à la formule (I),
R-X-A (I)
dans laquelle
A représente une chaîne composée de une à vingt unités carbohydrate, comprenant chacune 3 à 6 atomes de carbone, reliées entre elles, chacune de ces unités pouvant être éventuellement substituée par un halogène, par une fonction amine, une fonction acide, une fonction ester, un thiol, une fonction alcoxy, une fonction thio-éther, une fonction thio-ester, une fonction amide, une fonction carbamate, une fonction urée,
R représente une chaîne alcoyle ou une chaîne alcényle, comprenant de 4 à 24 atomes de carbone, ramifiée ou linéaire, pouvant être interrompue par des ponts éthers, portant éventuellement une fonction hydroxyle, une fonction acide carboxylique, une fonction amine, une fonction ester, une fonction acyloxy, une fonction amide, une fonction éther, une fonction carbamate, une fonction urée,
X représente une fonction susceptible de relier R et A,
en tant qu'agent desquamant de la peau.

3. Utilisation dans une composition topique, d'au moins un carbohydrate ou dérivé de carbohydrate répondant à la formule (I'),
R-X-A (I')
dans laquelle
les unités composant A sont choisies parmi la N-acétyl-D-glucosamine, l'acide N-acétyl-neuraminique, l'adonitol, le β-D-allose, le D-altrose, le 6-amino-6-déoxy-D-glucose, le 1,6-anhydroglucose, l'acide arabinique, le D-arabinose, le L-arabinose, le D,L-arabinose, le D-arabitol, le L-arabitol, le D-cellobiose, la D-glucosamine, le 2-déoxy-D-glucose, le mésoérythritol, le D-érythrose, le D-fructose, le D-fucose, le L-fucose, la glucamine, l'acide D-glucarique, le D-glucono-1,5-lactone, le L-glucono-1,5-lactone, la D-glucosamine, l'acide D-glucosaminique, l'acide D-glucuronique, le L-glucose, le D-glucose, l'isomaltitol, l'isomaltotriose, l'isomaltose, le D-lyxose, le L-lyxose, la lyxosamine, le maltitol, le D-maltose, le maltotétraose, le maltotriitol, le maltotriose, la D-mannosamine, le D-mannose, le L-mannose, le D-mélézitose, le L-rhamnose, le D-ribose, le L-ribose, le D-ribulose, le rutinose, le D-saccharose, l'α-sophorose, le sorbitol, le D-tagatose, le D-talose, le D-thréose, le turanose, le D-xylitol, le D-xylose, le L-xylose et le D,L-xylose, chacune de ces unités pouvant être éventuellement substituée par un halogène, par une fonction amine, une fonction acide, une fonction ester, un thiol, une fonction alcoxy, une fonction thio-éther, une fonction thio-ester, une fonction amide, une fonction carbamate, une fonction urée,
R représente une chaîne alcoyle ou une chaîne alcényle, comprenant de 4 à 24 atomes de carbone, ramifiée ou linéaire, pouvant être interrompue par des ponts éthers, portant éventuellement une fonction hydroxyle, une fonction acide carboxylique, une fonction amine, une fonction ester, une fonction acyloxy, une fonction amide, une fonction éther, une fonction carbamate, une fonction urée,
X représente une fonction susceptible de relier R et A,
en tant qu'agent pour traiter le vieillissement cutané.

4. Utilisation selon l'une quelconque des revendications 1 et 3, **caractérisée en ce que** X est une fonction amine, éther, amide, ester, urée, carbamate, thioester, thioéther, sulfonamide.

5. Utilisation selon l'une quelconque des revendications 1 à 4, **caractérisée en ce que** les unités composant A sont reliées entre elles par des ponts acétals.

6. Utilisation selon l'une quelconque des revendications 1 à 5, **caractérisée en ce que** chacune des unités composant A est un sucre ou un dérivé de sucre.

7. Utilisation selon l'une quelconque des revendications 1 à 6, **caractérisée en ce qu'**au moins une des unités composant A est choisie parmi les sucres réduits, les sucres aminés et les sucres porteurs d'une fonction acide carboxylique.

8. Utilisation selon l'une quelconque des revendications 1 à 7, **caractérisée en ce que** R représente une chaîne alcoyle ou une chaîne alcényle, comprenant de 4 à 24 atomes de carbone, ramifiée ou linéaire, portant une fonction hydroxyle.

9. Utilisation selon la revendication 1, **caractérisée en ce que** les unités composant A sont choisies parmi : la N-acétyl-D-galactosamine, la N-acétyl-D-glucosamine, l'acide N-acétyl-neuraminique, l'adonitol, le β-D-allose, le D-altrose, le 6-amino-6-déoxy-D-glucose, le 1,6-anhydroglucose, l'acide arabinique, l'arabinogalactan, le D-arabinose, le L-arabinose, le D,L-arabinose, le D-arabitol, le L-arabitol, le D-cellobiose, la D-glucosamine, la D-galactosamine, le 2-déoxy-D-glucose, le 6-déoxy-D-galactose, le 6-déoxy-L-galactose, le galactitol, le mésoérythritol, le D-érythrose, le D-fructose, le D-fucose, le L-fucose, l'acide D-galactarique, le galactitol, le galactomannane, le D-galactono-1,4-lactone, le L-galactono-1,4-lactone, la D-galactosamine, le D-galactose, le L-galactose, l'acide D-galacturonique, le β-gentiobiose, la glucamine, l'acide D-glucarique, l'acide D-glucarique, le D-glucono-1,5-lactone, le L-glucono-1,5-lactone, la D-glucosamine, l'acide D-glucosaminique, l'acide D-glucuronique, le L-glucose, le D-glucose, l'isomaltitol, l'isomaltotriose, l'isomaltose, l'acide lactobionique, le D-lactose, le lactulose, le D-lyxose, le L-lyxose, la lyxosamine, le maltitol, le D-maltose, le maltotétraose, le maltotriitol, le maltotriose, la D-mannosamine, le D-mannose, le L-mannose, le D-mélézitose, le D-mélibiose, le D-raffinose, l'undeca-acétate de D-raffinose, le L-rhamnose, le D-ribose, le L-ribose, le D-ribulose, le rutinose, le D-saccharose, l'α-sophorose, le sorbitol, le D-tagatose, le D-talose, le D-thréose, le turanose, le D-xylitol, le D-xylose, le L-xylose et le D,L-xylose.

10. Utilisation selon l'une quelconque des revendications 1 à 9, **caractérisée en ce que** A est choisi parmi :
la D-glucosamine, la D-glucamine, la N-méthyl-D-glucamine, le D-glucose, le D-maltose, le sorbitol et le maltitol.

11. Utilisation selon l'une quelconque des revendications 1 à 10, **caractérisée en ce que** R comprend 4 à 16 atomes de carbone.

12. Utilisation selon l'une quelconque des revendications 1 à 11, **caractérisée en ce que** (I) est choisi parmi :
le N-butanoyl-D-glucosamine, le N-octanoyl-D-glucosamine, le N-octyloxycarbonyl-N-méthyl-D-glucamine, le N-2-éthyl-hexyloxycarbonyl-N-méthyl-D-glucamine, le 6-O-octanoyl-D-glucose, le 6'-O-octanoyl-D-maltose, le 6'-O-dodecanoyl-D-maltose.

13. Utilisation selon l'une quelconque des revendications 1 à 12, **caractérisée en ce que** le carbohydrate selon (I) ou le mélange de carbohydrates selon (I) est présent en une quantité allant de 0,05 à 20% en poids par rapport au poids total de la composition

14. Utilisation selon l'une quelconque des revendications 1 à 13, **caractérisée en ce que** le carbohydrate selon (I) ou le mélange de carbohydrates selon (I) est présent en une quantité allant de 0,2 à 10% et de préférence de 0,5 à 5% en poids par rapport au poids total de la composition.

15. Utilisation selon l'une quelconque des revendications 1 à 14, **caractérisée en ce que** la composition topique comprend, de plus, au moins un autre actif ayant des propriétés desquamantes.

16. Utilisation selon l'une quelconque des revendications 1 à 15, **caractérisée en ce que** la composition topique comprend, en outre, des α-hydroxyacides ou des β -hydroxyacides, qui peuvent être linéaires, ramifiés ou cycliques, saturés ou insaturés, les atomes d'hydrogène de la chaîne carbonée pouvant, être substitués par des halogènes, des radicaux halogénés, alkylés, acylés, acyloxylés, alcoxy carbonylés ou alcoxylés ayant de 2 à 18 atomes de carbone.

17. Utilisation selon l'une quelconque des revendications 1 à 16, **caractérisée en ce que** la composition comprend également au moins un produit choisi parmi : les acides de fruits, l'acide salicylique, ainsi que ses dérivés alkylés, acylés ou alcoxylés, l'acide rétinoïque (all-trans ou 13-cis) et ses dérivés, le rétinol (vitamine A) et ses esters ainsi que leurs sels, le rétinal.

18. Utilisation selon l'une quelconque des revendications 1 à 17, **caractérisée en ce que** la composition comprend également au moins un produit choisi parmi :
les acides glycolique, lactique, malique, tartrique, citrique, mandélique, salicylique, l'acide n-octanoyl-5-salicylique, l'acide n-dodécanoyl-5-salicylique, l'acide n-décanoyl-5-salicylique, l'acide n-octyl-5-salicylique, l'acide n-heptyloxy-5 ou -4-salicylique, l'acide 2-hydroxy-3-méthyl-benzoïque, l'acide 2-hydroxy-3-méthoxybenzoïque, l'acide rétinoïque (all-trans ou 13-cis), le rétinol (vitamine A), le palmitate de rétinol, l'acétate de rétinol, le propionate de rétinol ainsi que leurs sels et le rétinal.

19. Utilisation selon l'une quelconque des revendications 1 à 18, **caractérisée en ce que** la composition comprend, outre le ou les carbohydrates selon (I), au moins un autre composé à propriété desquamante, représentant de 0,01 à 5 % en poids du poids total de la composition et mieux de 0,1 à 3 %.

20. Utilisation selon l'une quelconque des revendications 1 à 19, **caractérisée en ce que** la composition comprend également au moins un filtre solaire complémentaire, actif dans l'UVA et/ou l'UVB, hydrophile ou lipophile, et comportant éventuellement une fonction sulfonique.

21. Utilisation selon l'une quelconque des revendications 1 à 20, **caractérisée en ce que** la composition comprend également au moins un antagoniste de substance P et/ou de CGRP.

22. Utilisation selon l'une quelconque des revendications 1 à 21, **caractérisée en ce que** la composition comprend également au moins un produit choisi parmi l'Iris Pallida, les sels de strontium, notamment les chlorures et les nitrates de strontium.

## Claims

1. Use for the production of a topical composition, intended for promoting the desquamation of the skin, of at least one carbohydrate or carbohydrate derivative corresponding to the formula (I),
R-X-A (I)
in which
A is a chain formed of one to twenty carbohydrate units, each comprising 3 to 6 carbon atoms, connected to each other, it being possible for each of these units to be optionally substituted by a halogen, by an amine function, an acid function, an ester function, a thiol, an alkoxy function, a thioether function, a thioester function, an amide function, a carbamate function or a urea function,
R is an alkyl chain or an alkenyl chain, comprising from 4 to 24 carbon atoms, which is branched or linear, it being possible for it to be interrupted by ether bridges, optionally carrying a hydroxyl function, a carboxylic acid function, an amine function, an ester function, an acyloxy function, an amide function, an ether function, a carbamate function or a urea function,
X is a function capable of connecting R and A.

2. Use in a topical composition, of at least one carbohydrate or carbohydrate derivative corresponding to the formula (I),
R-X-A (I)
in which
A is a chain formed of one to twenty carbohydrate units, each comprising 3 to 6 carbon atoms, connected to each other, it being possible for each of these units to be optionally substituted by a halogen, by an amine function, an acid function, an ester function, a thiol, an alkoxy function, a thioether function, a thioester function, an amide function, a carbamate function or a urea function,
R is an alkyl chain or an alkenyl chain, comprising from 4 to 24 carbon atoms, which is branched or linear, it being possible for it to be interrupted by ether bridges, optionally carrying a hydroxyl function, a carboxylic acid function, an amine function, an ester function, an acyloxy function, an amide function, an ether function, a carbamate function or a urea function,
X is a function capable of connecting R and A, as a desquamating agent of the skin.

3. Use in a topical composition, of at least one carbohydrate or carbohydrate derivative corresponding to the formula (I'),
R-X-A (I')
in which
the units forming A are chosen from: N-acetyl-D-glucosamine, N-acetylneuraminic acid, adonitol, β-D-allose, D-altrose, 6-amino-6-deoxy-D-glucose, 1,6-anhydro-glucose, arabinic acid, D-arabinose, L-arabinose, D,L-arabinose, D-arabitol, L-arabitol, D-cellobiose, D-glucosamine, 2-deoxy-D-glucose, mesoerythritol, D-erythrose, D-fructose, D-fucose, L-fucose, glucamine, D-glucaric acid, D-glucono-1,5-lactone, L-glucono-1,5-lactone, D-glucosamine, D-glucosaminic acid, D-glucuronic acid, L-glucose, D-glucose, isomaltitol, isomaltotriose, isomaltose, D-lyxose, L-lyxose, lyxosamine, maltitol, D-maltose, maltotetraose, maltotriitol, maltotriose, D-mannosamine, D-mannose, L-mannose, D-melezitose, L-rhamnose, D-ribose, L-ribose, D-ribulose, rutinose, D-sucrose, α-sophorose, sorbitol, D-tagatose, D-talose, D-threose, turanose, D-xylitol, D-xylose, L-xylose and D,L-xylose, it being possible for each of these units to be optionally substituted by a halogen, by an amine function, an acid function, an ester function, a thiol, an alkoxy function, a thioether function, a thioester function, an amide function, a carbamate function or a urea function,
R is an alkyl chain or an alkenyl chain, comprising from 4 to 24 carbon atoms, which is branched or linear, it being possible for it to be interrupted by ether bridges, optionally carrying a hydroxyl function, a carboxylic acid function, an amine function, an ester function, an acyloxy function, an amide function, an ether function, a carbamate function or a urea function,
X is a function capable of connecting R and A, as an agent against cutaneous ageing.

4. Use according to either of Claims 1 to 3, **characterized in that** X is an amine, ether, amide, ester, urea, carbamate, thioester, thioether or sulphonamide function.

5. Use according to any one of Claims 1 to 4, **characterized in that** the units forming A are connected to each other by acetal bridges.

6. Use according to any one of Claims 1 to 5, **characterized in that** each of the units forming A is a sugar or a sugar derivative.

7. Use according to any one of Claims 1 to 6, **characterized in that** at least one of the units forming A is chosen from reduced sugars, amino sugars and sugars carrying a carboxylic acid function.

8. Use according to any one of Claims 1 to 7, **characterized in that** R is an alkyl chain or an alkenyl chain, comprising from 4 to 24 carbon atoms, which is branched or linear, carrying a hydroxyl function.

9. Use according to Claim 1, **characterized in that** the units forming A are chosen from: N-acetyl-D-galactosamine, N-acetyl-D-glucosamine, N-acetylneuraminic acid, adonitol, β-D-allose, D-altrose, 6-amino-6-deoxy-D-glucose, 1,6-anhydro-glucose, arabinic acid, arabinogalactan, D-arabinose, L-arabinose, D,L-arabinose, D-arabitol, L-arabitol, D-cellobiose, D-glucosamine, D-galactosamine, 2-deoxy-D-glucose, 6-deoxy-D-galactose, 6-deoxy-L-galactose, galactitol, mesoerythritol, D-erythrose, D-fructose, D-fucose, L-fucose, D-galactaric acid, galactitol, galactomannan, D-galactono-1,4-lactone, L-galactono-1,4-lactone, D-galactosamine, D-galactose, L-galactose, D-galacturonic acid, β-gentiobiose, glucamine, D-glucaric acid, D-glucono-1,5-lactone, L-glucono-1,5-lactone, D-glucosamine, D-glucosaminic acid, D-glucuronic acid, L-glucose, D-glucose, isomaltitol, isomaltotriose, isomaltose, lactobionic acid, D-lactose, lactulose, D-lyxose, L-lyxose, lyxosamine, maltitol, D-maltose, maltotetraose, maltotriitol, maltotriose, D-mannosamine, D-mannose, L-mannose, D-melezitose, D-melibiose, D-raffinose, D-raffinose undecaacetate, L-rhamnose, D-ribose, L-ribose, D-ribulose, rutinose, D-sucrose, α-sophorose, sorbitol, D-tagatose, D-talose, D-threose, turanose, D-xylitol, D-xylose, L-xylose and D,L-xylose.

10. Use according to any one of Claims 1 to 9, **characterized in that** A is chosen from:
D-glucosamine, D-glucamine, N-methyl-D-glucamine, D-glucose, D-maltose, sorbitol and maltitol.

11. Use according to any one of Claims 1 to 10, **characterized in that** R comprises 4 to 16 carbon atoms.

12. Use according to any one of Claims 1 to 11, **characterized in that** (I) is chosen from:
N-butanoyl-D-glucosamine, N-octanoyl-D-glucosamine, N-octyloxycarbonyl-N-methyl-D-glucamine, N-2-ethylhexyloxycarbonyl-N-methyl-D-glucamine, 6-O-octanoyl-D-glucose, 6'-O-octanoyl-D-maltose, 6'-O-dodecanoyl-D-maltose.

13. Use according to any one of Claims 1 to 12, **characterized in that** the carbohydrate according to (I) or the mixture of carbohydrates according to (I) is present in a quantity ranging from 0.05 to 20% by weight with respect to the total weight of the composition.

14. Use according to any one of Claims 1 to 13, **characterized in that** the carbohydrate according to (I) or the mixture of carbohydrates according to (I) is present in a quantity ranging from 0.2 to 10% and preferably from 0.5 to 5% by weight with respect to the total weight of the composition.

15. Use according to any one of Claims 1 to 14, **characterized in that** the topical composition comprises, in addition, at least one other active agent having desquamating properties.

16. Use according to any one of Claims 1 to 15, **characterized in that** the topical composition comprises, in addition, α-hydroxy acids or β-hydroxy acids, which can be linear, branched or cyclic, saturated or unsaturated, it being possible for the hydrogen atoms of the carbon chain to be substituted by halogens, or halogenated, alkylated, acylated, acyloxylated, alkoxycarbonylated or alkoxylated radicals having from 2 to 18 carbon atoms.

17. Use according to any one of Claims 1 to 16, **characterized in that** the composition likewise comprises at least one product chosen from:
fruit acids, salicylic acid, as well as its alkylated, acylated or alkoxylated derivatives, retinoic acid (all-trans or 13-cis) and its derivatives, retinol (vitamin A) and its esters as well as their salts, and retinal.

18. Use according to any one of Claims 1 to 17, **characterized in that** the composition likewise comprises at least one product chosen from:
glycolic, lactic, malic, tartaric, citric, mandelic or salicylic acid, 5-n-octanoylsalicylic acid, 5-n-dodecanoylsalicylic acid, 5-n-decanoylsalicylic acid, 5-n-octylsalicylic acid, 5- or 4-n-heptyloxysalicylic acid, 2-hydroxy-3-methylbenzoic acid, 2-hydroxy-3-methoxybenzoic acid, retinoic acid (all-trans or 13-cis), retinol (vitamin A), retinol palmitate, retinol acetate, retinol propionate as well as their salts, and retinal.

19. Use according to any one of Claims 1 to 18, **characterized in that** the composition comprises, apart from the carbohydrate(s) according to (I), at least one other compound with desquamating properties, representing from 0.01 to 5% by weight of the total weight of the composition and, better, from 0.1 to 3%.

20. Use according to any one of Claims 1 to 19, **characterized in that** the composition likewise comprises at least one hydrophilic or lipophilic complementary solar filter, which is active in UVA and/or UVB, and optionally comprising a sulphonic function.

21. Use according to any one of Claims 1 to 20, **characterized in that** the composition likewise comprises at least one antagonist of substance P and/or of CGRP.

22. Use according to any one of Claims 1 to 21, **characterized in that** the composition likewise comprises at least one product chosen from Iris pallida, and strontium salts, especially the chlorides and nitrates of strontium.

## Patentansprüche

1. Verwendung mindestens eines Kohlenhydrats oder Kohlenhydratderivats der Formel (1) zur Herstellung einer topischen Zusammensetzung:
R-X-A (I),
worin bedeuten:
A eine Kette, die sich aus einer bis zwanzig Kohlenhydrateinheiten zusammensetzt, die jeweils 3 bis 6 Kohlenstoffatome enthalten und miteinander verbunden sind, wobei jede Einheit ggf. mit einem Halogen, einer Aminogruppe, einer Säuregruppe, einer Estergruppe, einem Thiol, einer Alkoxygruppe, einer Thioethergruppe, einer Thioestergruppe, einer Amidgruppe, einer Carbamatgruppe oder einer Harnstoffgruppe substituiert sein kann,
R eine geradkettige oder verzweigte Alkyl- oder Alkenylgruppe mit 4 bis 24 Kohlenstoffatomen, die durch Etherbrücken unterbrochen sein kann und ggf. eine Hydroxygruppe, Carbonsäuregruppe, Aminogruppe, Estergruppe, Acyloxygruppe, Amidgruppe, Ethergruppe, Carbamatgruppe oder Harnstoffgruppe trägt, und
X eine Gruppe, die R und A miteinander verbinden kann,
bestimmt für die Abschuppung der Haut zu fördern.

2. Verwendung mindestens eines Kohlenhydrats oder Kohlenhydratderivats der Formel (I) in einer topischen Zusammensetzung:
R-X-A (I),
worin bedeuten:
A eine Kette, die sich aus einer bis zwanzig Kohlenhydrateinheiten zusammensetzt, die jeweils 3 bis 6 Kohlenstoffatome enthalten und miteinander verbunden sind, wobei jede Einheit ggf. mit einem Halogen, einer Aminogruppe, einer Säuregruppe, einer Estergruppe, einem Thiol, einer Alkoxygruppe, einer Thioethergruppe, einer Thioestergruppe, einer Amidgruppe, einer Carbamatgruppe oder einer Harnstoffgruppe substituiert sein kann,
R eine geradkettige oder verzweigte Alkyl- oder Alkenylgruppe mit 4 bis 24 Kohlenstoffatomen, die durch Etherbrücken unterbrochen sein kann und ggf. eine Hydroxygruppe, Carbonsäuregruppe, Aminogruppe, Estergruppe, Acyloxygruppe, Amidgruppe, Ethergruppe, Carbamatgruppe oder Harnstoffgruppe trägt, und
X eine Gruppe, die R und A miteinander verbinden kann,
als wirkstoff für die Abschuppung der Haut.

3. Verwendung mindestens eines Kohlenhydrats oder Kohlenhydratderivats der Formel (I') in einer topischen Zusammensetzung:
R-X-A (I'),
worin die Einheiten, die die Gruppe A bilden, ausgewählt sind unter: N-Acetyl-D-glucosamin, N-Acetyl-neuraminsäure, Adonit, β-D-Allose, D-Altrose, 6-Amino-6-deoxy-D-glucose, 1,6-Anhydroglucose, Arabinsäure, D-Arabinose, L-Arabinose, D,L-Arabinose, D-Arabit, L-Arabit, D-Cellobiose, D-Glucosamin, 2-Deoxy-D-glucose, Mesoerythrit, D-Erythrose, D-Fructose, D-Fucose, L-Fucose, Glucamin, D-Glucarsäure, D-Glucono-1,5-lacton, L-Glucono-1,5-lacton, D-Glucosamin, D-Glucosaminsäure, D-Glucuronsäure, L-Glucose, D-Glucose, Isomaltit, Isomaltotriose, Isomaltose, D-Lyxose, L-Lyxose, Lyxosamin, Maltit, D-Maltose, Maltotetraose, Maltotriitol, Maltotriose, D-Mannosamin, D-Mannose, L-Mannose, D-Melezitose, L-Rhamnose, D-Ribose, L-Ribose, D-Ribulose, Rutinose, D-Saccharose, α-Sophorose, Sorbit, D-Tagatose, D-Talose, D-Threose, Turanose, D-Xylit, D-Xylose, L-Xylose und D,L-Xylose, wobei jede Einheit ggf. mit einem Halogen, einer Aminogruppe, einer Säuregruppe, einer Estergruppe, einem Thiol, einer Alkoxygruppe, einer Thioethergruppe, einer Thioestergruppe, einer Amidgruppe, einer Carbamatgruppe und einer Harnstoffgruppe substituiert sein kann,
R eine geradkettige oder verzweigte Alkyl- oder Alkenylgruppe mit 4 bis 24 Kohlenstoffatomen bedeutet, die durch Etherbrücken unterbrochen sein kann und ggf. eine Hydroxygruppe, Carbonsäuregruppe, Aminogruppe, Estergruppe, Acyloxygruppe, Amidgruppe, Ethergruppe, Carbamatgruppe oder Harnstoffgruppe enthalten kann, und
X eine Gruppe, die R und A miteinander verbinden kann,
als wirkstoff zur Behandlung der Hautalterung.

4. Verwendung nach einem der Ansprüche 1 bis 3, **dadurch gekennzeichnet, dass** X Amin, Ether; Amid, Ester, Harnstoff, Carbamat, Thioester, Thioether oder Sulfonamid bedeutet.

5. Verwendung nach einem der Ansprüche 1 bis 4, **dadurch gekennzeichnet, dass** die Einheiten, die A bilden, über Acetalbrücken miteinander verbunden sind.

6. Verwendung nach einem der Ansprüche 1 bis 5 **dadurch gekennzeichnet, dass** die Einheiten, die A bilden, Zucker oder Zuckerderivate sind.

7. Verwendung nach einem der Ansprüche 1 bis 6, **dadurch gekennzeichnet, dass** mindestens eine der Einheiten, die A bilden, unter den reduzierten Zuckern, Aminozuckern und Zuckern mit Carbonsäuregruppe ausgewählt sind.

8. Verwendung nach einem der Ansprüche 1 bis 7, **dadurch gekennzeichnet, dass** R eine geradkettige oder verzweigte Alkyl- oder Alkenylgruppe mit 4 bis 24 Kohlenstoffatomen bedeutet, die eine Hydroxygruppe trägt.

9. Verwendung nach Anspruch 1, **dadurch gekennzeichnet, dass** die Einheiten, die die Gruppe A bilden, ausgewählt sind unter: N-Acetyl-D-galactosamin, N-Acetyl-D-glucosamin, N-Acetyl-neuraminsäure, Adonit, β-D-Allose, D-Altrose, 6-Amino-6-deoxy-Dglucose, 1,6-Anhydroglucose, Arabinsäure, Arabinogalactan, D-Arabinose, L-Arabinose, D,L-Arabinose, D-Arabit, L-Arabit, D-Cellobiose, D-Glucosamin, D-Galactosamin, 2-Deoxy-D-glucose, 6-Deoxy-D-galactose, 6-Deoxy-L-galactose, Galactit, Mesoerythrit, D-Erythrose, D-Fructose, D-Fucose, L-Fucose, D-Galactarsäure, Galactit, Galactomannan, D-Galactono-1,4-lacton, L-Galactono-1,4-lacton, D-Galactosamin, D-Galactose, L-Galactose, D-Galacturonsäure, β-Gentiobiose, Glucamin, D-Glucarsäure, D-Glucono,1,5-lacton, L-Glucono-1,5-lacton, D-Glucosamin, D-Glucosaminsäure, D-Glucuronsäure, L-Glucose, D-Glucose, Isomaltit, Isomaltotriose, Isomaltose, Lactobionsäure, D-Lactose, Lactulose, D-Lyxose, L-Lyxose, Lyxosamin, Maltit, D-Maltose, Maltotetraose, Maltotriitol, Maltotriose, D-Mannosamin, D-Mannose, L-Mannose, D-Melezitose, D-Melibiose, D-Raffinose, D-Raffinoseundecaacetat, L-Rhamnose, D-Ribose, L-Ribose, D-Ribulose, Rutinose, D-Saccharose, α-Sophorose, Sorbit, D-Tagatose, D-Talose, D-Threose, Turanose, D-Xylit, D-Xylose, L-Xylose und D,L-Xylose.

10. Verwendung nach einem der Ansprüche 1 bis 9, **dadurch gekennzeichnet, dass** A ausgewählt ist unter: D-Glucosamin, D-Glucamin, N-Methyl-D-glucamin, D-Glucose, D-Maltose, Sorbit und Maltit.

11. Verwendung nach einem der Ansprüche 1 bis 10, **dadurch gekennzeichnet, dass** R 4 bis 16 Kohlenstoffatome aufweist.

12. Verwendung nach einem der Ansprüche 1 bis 11, **dadurch gekennzeichnet, dass** (I) ausgewählt ist unter: N-Butanoyl-D-glucosamin, N-Octanoyl-D-glucosamin, N-Octyloxycarbonyl-N-methyl-Dglucamin, N-2-Ethylhexyloxycarbonyl-N-methyl-D-glucamin, 6-O-Octanoyl-D-glucose, 6'-O-Octanoyl-D-maltose und 6'-O-Dodecanoyl-D-maltose.

13. Verwendung nach einem der Ansprüche 1 bis 12, **dadurch gekennzeichnet, dass** das Kohlenhydrat (I) oder Gemisch von Kohlenhydraten (I) in einer Menge von 0,05 bis 20 Gew.-%, bezogen auf das Gesamtgewicht der Zusammensetzung, vorliegt.

14. Verwendung nach einem der Ansprüche 1 bis 13, **dadurch gekennzeichnet, dass** das Kohlenhydrat (I) oder das Gemisch von Kohlenhydraten (I) in einer Menge von 0,2 bis 10% und vorzugsweise 0,5 bis 5 Gew.-%, bezogen auf das Gesamtgewicht der Zusammensetzung, vorliegt.

15. Verwendung nach einem der Ansprüche 1 bis 14, **dadurch gekennzeichnet, dass** die Zusammensetzung zur topischen Anwendung ferner mindestens einen Wirkstoff mit abschuppenden Eigenschaften enthält.

16. Verwendung nach einem der Ansprüche 1 bis 15, **dadurch gekennzeichnet, dass** die Zusammensetzung zur topischen Anwendung ferner α-Hydroxysäuren oder β-Hydroxysäuren enthält, die geradkettig, verzweigt oder cyclisch, gesättigt oder ungesättigt vorliegen können, wobei die Wasserstoffatome der Kohlenstoffkette durch Halogene, halogenierte Gruppen, Alkylgruppen, Acylgruppen, Acyloxygruppen, Alkoxycarbonylgruppen oder Alkoxygruppen mit 2 bis 18 Kohlenstoffatomen ersetzt sein können.

17. Verwendung nach einem der Ansprüche 1 bis 16, **dadurch gekennzeichnet, dass** die Zusammensetzung außerdem mindestens ein Produkt enthält, das unter den Fruchtsäuren, Salicylsäure sowie ihren Alkyl-, Acyl- oder Alkoxyderivaten, Retinsäure (all trans oder 13-cis) und ihren Derivaten, Retinol (Vitamin A) und seinen Estern sowie deren Salzen und Retinal ausgewählt ist.

18. Verwendung nach einem der Ansprüche 1 bis 17, **dadurch gekennzeichnet, dass** die Zusammensetzung außerdem mindestens ein Produkt enthält, das ausgewählt ist unter: Glykolsäure, Milchsäure, Äpfelsäure, Weinsäure, Citronensäure, Mandelsäure, Salicylsäure, 5-n-Octanoylsalicylsäure, 5-n-Dodecanoylsalicylsäure, 5-n-Decanoylsalicylsäure, 5-n-Octylsalicylsäure, 5-n-Heptyloxysalicylsäure, 4-N-Heptyloxysalicylsäure, 2-Hydroxy-3-methylbenzoesäure, 2-Hydroxy-3-methoxybenzoesäure, Retinsäure (all trans oder 13-cis), Retinol (Vitamin A), Retinolpalmitat, Retinolacetat, Retinolpropionat oder deren Salzen und Retinal.

19. Verwendung nach einem der Ansprüche 1 bis 18, **dadurch gekennzeichnet, dass** die Zusammensetzung neben dem Kohlenhydrat (I) oder den Kohlenhydraten (I) außerdem mindestens eine weitere Verbindung mit abschuppenden Eigenschaften enthält, die in einer Menge von 0,01 bis 5 Gew.-% des Gesamtgewichts der Zusammensetzung und besser 0,1 bis 3% vorliegt.

20. Verwendung nach einem der Ansprüche 1 bis 19, **dadurch gekennzeichnet, dass** die Zusammensetzung außerdem mindestens ein zusätzliches, im UVA- und/oder UVB-Bereich wirksames, hydrophiles oder lypophiles Sonnenschutzfilter enthält, das ggf. eine Sulfonsäuregruppe trägt.

21. Verwendung nach einem der Ansprüche 1 bis 20 **dadurch gekennzeichnet, dass** die Zusammensetzung ferner mindestens einen Antagonisten der Substanz P und/oder CGRP-Antagonisten enthält.

22. Verwendung nach einem der Ansprüche 1 bis 21, **dadurch gekennzeichnet, dass** die Zusammensetzung ferner mindestens ein Produkt enthält, das unter Iris Pallida, Strontiumsalzen und insbesondere Strontiumchloriden und Strontiumnitraten ausgewählt ist.
